# EUROPEAN PATENT SPECIFICATION

(11) **EP 1 543 144 B1**
(45) Date of publication and mention of the grant of the patent: **02.05.2007**
(21) Application number: 03798174.3
(22) Date of filing: 23.09.2003
(51) Int. Cl.: C12P 23/00

(54) **PRODUCTION OF ZEAXANTHIN AND BETA-CRYPTOXANTHIN BY PHAFFIA**
PRODUKTION VON ZEAXANTHIN UND BETA-CRYPTOXANTHIN DURCH PHAFFIA
PRODUCTION DE ZEAXANTHINE ET BETA-CRYPTOXANTHINE PAR PHAFFIA

(30) Priority: 27.09.2002 EP 02021599
(43) Date of publication of application: 22.06.2005
(73) Proprietor: DSM IP Assets B.V., 6411 TE Heerlen (NL)
(72) Inventor: HOSHINO, Tatsuo, Kanagawa-ken, Kanagawa 248-0027 (JP); OJIMA, Kazuyuki, Kanagawa-ken, Kanagawa 251-0877 (JP); SETOGUCHI, Yutaka, Kanagawa-ken, Kanagawa 251-0033 (JP)
(74) Representative: Keller, Günter
(86) International application number: PCT/EP2003/010574
(87) International publication number: WO 2004/029275

(56) References cited:
- EP-A- 0 747 483
- EP-A- 0 933 427
- WO-A-94/06918
- US-A- 5 744 341
- US-A- 6 124 113
- US-B1- 6 291 204

### SEQUENCE LISTING

<110> Roche vitamins AG
<120> Production of zeaxanthin by Phaffia
<130> NDR5225
<160> 10
<170> PatentIn version 3.1
<210> 1
   <211> 26
   <212> DNA
   <213> Artificial
<400> 1
   cccgggatga gcacttgggc cgcaat 26
<210> 2
   <211> 26
   <212> DNA
   <213> Artificial
<400> 2
   ggatcctcat gtattgcgat ccgccc 26
<210> 3
   <211> 26
   <212> DNA
   <213> Artificial
<400> 3
   cccgggatgc tagtaaatag tttaat 26
<210> 4
   <211> 26
   <212> DNA
   <213> Artificial
<400> 4
   gtcgacttat tcgggcgaag acgacg 26
<210> 5
   <211> 28
   <212> DNA
   <213> Artificial
<400> 5
   gcggccgctg gtgggtgcat gtatgtac 28
<210> 6
   <211> 26
   <212> DNA
   <213> Artificial
<400> 6
   cccggggatg gtaagagtgt tagaga 26
<210> 7
   <211> 33
   <212> DNA
   <213> Artificial
<400> 7
   ggatccgtcg actaaacggt tctctccaaa ccc 33
<210> 8
   <211> 28
   <212> DNA
   <213> Artificial
<400> 8
   ggtaccttga tcagataaag atagagat 28
<210> 9
   <211> 20
   <212> DNA
   <213> Artificial
<400> 9
   gagctctcga gtggacggtg 20
<210> 10
   <211> 28
   <212> DNA
   <213> Artificial
<400> 10
   gcggccgcga gctcatcccg cttcactc 28

### SEQUENCE LISTING

<110> Roche vitamins AG
<120> Production of zeaxanthin by Phaffia
<130> NDR5225-case 21415
<140> PCT/EP03/10574
   <141> 2003-09-23
<150> EP 02021599.2
   <151> 2002-09-27
<160> 10
<170> PatentIn version 3.1
<210> 1
   <211> 26
   <212> DNA
   <213> Artificial sequence
<220>
   <223> Primer PZ1
<400> 1
   cccgggatga gcacttgggc cgcaat 26
<210> 2
   <211> 26
   <212> DNA
   <213> Artificial Sequence
<220>
   <223> Primer PZ2
<400> 2
   ggatcctcat gtattgcgat ccgccc 26
<210> 3
   <211> 26
   <212> DNA
   <213> Artificial Sequence
<220>
   <223> Primer EZ1
<400> 3
   cccgggatgc tagtaaatag tttaat 26
<210> 4
   <211> 26
   <212> DNA
   <213> Artificial sequence
<220>
   <223> Primer EZ2
<400> 4
   gtcgacttat tcgggcgaag acgacg 26
<210> 5
   <211> 28
   <212> DNA
   <213> Artificial Sequence
<220>
   <223> Primer GAP#1
<400> 5
   gcggccgctg gtgggtgcat gtatgtac 28
<210> 6
   <211> 26
   <212> DNA
   <213> Artificial Sequence
<220>
   <223> Primer GAP#5
<400> 6
   cccggggatg gtaagagtgt tagaga 26
<210> 7
   <211> 33
   <212> DNA
   <213> Artificial Sequence
<220>
   <223> Primer GAP#33
<400> 7
   ggatccgtcg actaaacggt tctctccaaa ccc 33
<210> 8
   <211> 28
   <212> DNA
   <213> Artificial sequence
<220>
   <223> Primer GAP#4
<400> 8
   ggtaccttga tcagataaag atagagat 28
<210> 9
   <211> 20
   <212> DNA
   <213> Artificial Sequence
<220>
   <223> Primer R#1
<400> 9
   gagctctcga gtggacggtg 20
<210> 10
   <211> 28
   <212> DNA
   <213> Artificial Sequence
<220>
   <223> Primer R#2
<400> 10
   gcggccgcga gctcatcccg cttcactc 28

## Description

The present invention relates to production of xanthophyll carotenoids, especially zeaxanthin and β-cryptoxanthin by microorganism belonging to genus Phaffia.

WO 94/06918 discloses a method for transforming a Phaffia strain which makes possible the cloning and expression of cloned heterologous or homologous genes.

US 5,744,341 discloses DNA and amino acid sequences for β-carotene hydroxylase.

More particularly, the present invention provides a process for producing xanthophyll carotenoids, especially zeaxanthin and β-cryptoxanthin by genetically modified recombinant microorganism belonging to genus *Phaffia*.

By using methods of the present invention, it becomes possible to produce the useful xanthophyll carotenoids other than astaxanthin such as zeaxanthin and β-cryptoxanthin as a major xanthophyll carotenoid in the recombinant microorganism belonging to the genus *Phaffia*.

Any strain which is able to produce β-carotene can be a suitable host strain. When the selected host strain is a normal *Phaffia* strain which is capable of producing astaxanthin from β-carotene, production of mixture of astaxanthin and other xanthophyll carotenoids can be achieved after introduction and expression of a gene coding for β-carotene hydroxylase. On the other hand, when a strain which can not produce astaxanthin and is accumulating β-carotene is selected as the host strain, it is expected to produce maximum level of xanthophyll carotenoids such as zeaxanthin and β-cryptoxanthin, without accumulation of astaxanthin. Such a host strain which accumulates β-carotene can be obtained by mutagenesis of a strain of *P. rhodozyma* accumulating astaxanthin.
Alternatively, such a host strain accumulating β-carotene can also be obtained by deactivating astaxanthin synthase which is an enzyme involved in the biosynthesis of astaxanthin from β-carotene and is disclosed in US 6,365,386. Disruption of the gene of astaxanthin synthase will be one of the most convenient ways to deactivate the enzyme.

Furthermore, such a host strain accumulating β-carotene can also be obtained from public type culture collections. E.g., *P. rhodozyma* ATCC96815 accumulating β-carotene can be purchased from American Type Culture Collection (P.O.Box 1549, Manassas, VA 20108, USA). Isolating a new β-carotene accumulating strain of *P. rhodozyma*, which may be a derivative or a spontaneous mutant of astaxanthin producing strain, from nature will be another approach to prepare the host strain of the present invention.

An aspect of the present invention is a process for producing zeaxanthin and β-cryptoxanthin which comprises cultivating a recombinant *Phaffia* strain which is expressing β-carotene hydroxylase.

Said β-carotene hydroxylase catalyzes hydroxylation of β-carotene at positions 3 and 3' on the β-ionone ring to produce the xanthophyll, zeaxanthin via β-cryptoxanthin. Genes encoding this enzyme have been isolated from several species, e.g., *crtZ* from *Flavobacterium* species (US 6,124,113), *crtZ* from *Erwinia uredovora, crtZ* from *Erwinia herbicola, crtZs* from marine bacteria, *Agrobacterium aurantiacum* and *Alcaligenes* sp., and *Paracoccus marcusii* (GenBank accession No. Y15112, 1997), and *Paracoccus carotinifaciens* sp.nov. β-carotene hydroxylase genes can also be obtained from plant species.

In the present invention, a: gene encoding a protein having the β-carotene hydroxylase activity can be used.

Said β-carotene hydroxylase gene can be obtained from *Flavobacterium* sp. R1534 WT (ATCC21588) (GenBank accession No. U62808), *E. uredovora* ATCC19321 (GenBank accession No. D90087), *E. herbicola* ATCC39368 (GenBank accession No. M87280), *Agrobacterium aurantiacum* (GenBank accession No. D58420), *Alcaligenes* PC-1 (GenBank accession No. D58422), *Paracoccus marcusii* MH1 (GenBank accession No. Y15112), or the gram-negative bacteria E-396 (FERM BP-4283) (JP-A Hei 10-155497) having the β-carotene hydroxylase gene.

Still more preferably, said β-carotene hydroxylase gene can be obtained from *Flavobacterium* sp. R1534 WT (ATCC21588) (GenBank accession No. U62808), or it can also be obtained as a DNA sequence which is substantially homologous thereto.

The expression "a DNA sequence which is substantially homologous" refers with respect to the DNA sequence encoding the β-carotene hydroxylase to a DNA sequence which encodes an amino acid sequence which shows more than 60%, preferably more than 70%, more preferably more than 80%, and most preferably more than 90% identical amino acids when compared to the amino acid sequence of crtZ of *Flavobacterium* sp. R1534 WT (ATCC21588) and is the amino acid sequence of a polypeptide which shows the same type of enzymatic activity as the enzyme encoded by crtZ of *Flavobacterium* sp. R1534 WT(ATCC21588).

By using the β-carotene hydroxylase gene, it is possible to render a microorganism belonging to the genus *Phaffia* an ability to produce zeaxanthin and β-cryptoxanthin. The recombinant microorganism of *Phaffia* expressing a β-carotene hydroxylase gene can be prepared by the well-known recombinant technology.

The techniques used to isolate or clone a DNA encoding β-carotene hydroxylase of the present invention are known in the art and include isolation from genomic DNA. The cloning of the DNA sequence of the present invention from such genomic DNA can be effected by using the polymerase chain reaction (hereinafter referred to as PCR).

The isolated or cloned DNA encoding β-carotene hydroxylase can be preferably utilized after cloning on a suitable expression vector for expression of the enzyme in the host microorganism *Phaffia*.

"Expression vector" includes vectors which are capable of expressing DNA sequences contained therein where such sequences are operably linked to other sequences such as control sequences which are capable of effecting the expression of said DNA sequences in a microorganism belonging to *Phaffia*. The term "operably linked" refers to a juxtaposition wherein the components so described are in a relationship permitting them to function in their intended manner. The term "control sequence" is intended to include, at a minimum, components which are necessary for expression of the gene of interest, and may also include additional advantageous components. Generally the control sequences include promoters, terminators and, in some instances, enhancers, transactivators, or transcription factors. Constitutive promoters, such as the glyceraldehyde-3-dehydrogenase (GAP) gene promoter derived from *P. rhodozyma* (WO 97/23,633), may be used to obtain constitutive expression. Inducible promoters can be also used in order to achieve an exactly-controlled expression. One of the examples for the inducible promoters is the promoter of genes encoding heat shock proteins or amylase gene, and the like.

Methods which are well known to those skilled in the art may be used to construct said expression vectors.

It is implied, although not explicitly stated, that expression vectors must be replicable in the host organisms either as episomes or as an integral part of a chromosomal DNA. Generally, higher stability of the gene may be expected in the latter case. To integrate the expression vector into the chromosome of the host microorganism by a homologous recombination, a vector is prepared to contain at least a portion of the DNA fragment whose sequence is homologous to the host genomic DNA. For this purpose, rDNA gene fragment can be effectively used in a microorganism belonging to *Phaffia*. The rDNA is a kind of satellite DNAs which exist in multicopies on the genome. By using the rDNA fragment as a targeting DNA on the expression vector, the objective DNA to be expressed on said vector can be integrated into the host genome, and also can exist as multicopies. This may give the gene dosage effects which will contribute to the overexpression of the objective enzymes. In the Examples of the present invention, such rDNA fragment was conveniently used for this purpose. The invention is intended to include other forms of expression vectors which serve equivalent functions and which are, or subsequently become, known.

An isolated DNA sequence encoding β-carotene hydroxylase may be manipulated in a variety of ways to provide for expression of the polypeptide. Manipulation of the nucleotide sequence encoding said β-carotene hydroxylase prior to its insertion into an expression vector may be desirable or necessary depending on the expression vector. The techniques for modifying nucleotide sequences utilizing cloning methods are well known in the art.

Recombinant DNA consisting of said gene of β-carotene hydroxylase which was cloned in the expression vector will be introduced into a host microorganism. Methods for the introduction of foreign DNA into fungal cells including a microorganism belonging to *Phaffia* are well known in the art. These include, e.g., transformation by the LiCl method, protoplast fusion, electroporation, particle-gun methods which comprises bombardment by particles coated with DNAs, and other methods known in the art. In the Examples of the present invention, the particle-gun method was applied as a transformation method for *P. rhodozyma*.

The recombinant organism thus obtained is capable of overexpressing the DNA sequence encoding β-carotene hydroxylase. Thus, the recombinant organism of the present invention is useful in the production process of xanthophyll carotenoids, especially zeaxanthin and β-cryptoxanthin.

A further aspect of the present invention is a biological process for producing zeaxanthin and β-cryptoxanthin which comprises cultivating the recombinant microorganism of *Phaffia* in the presence of substrate for producing carotenoids in an aqueous nutrient medium under aerobic conditions, and isolating the resulting carotenoids from the cells of said recombinant microorganism or from the cultured broth.

Carotenoids including the xanthophylls are normally produced by cultivating a strain of *Phaffia* in a medium which comprises suitable macro- and micronutrients for the cells, such as molasses, saccharose or glucose as a carbohydrate source for cell growth and also as a substrate for producing carotenoids, and nitrogen sources such as corn steep liquor, yeast extract, diammonium sulphate, ammonium phosphate, ammonium hydroxide or urea, phosphorus sources such as ammonium phosphate and phosphoric acid and added micronutrients or mineral salts such as magnesium sulphate, zinc sulphate and biotin or desthiobiotin.

The conditions for cultivation are in a pH range of from 4 to 8 and in a temperature range of from 15 to 26°C for 24 to 500 hours.

Preferable conditions for cultivation are a pH in the range of from 5 to 7 and in a temperature range of from 18 to 22°C for 48 to 350 hours.

In the cultivation, aeration and agitation usually give favorable results for the production of carotenoids.

Once carotenoids were produced by cultivating the recombinant strain of *Phaffia* using the methods of the present invention, the carotenoids can be isolated either from the medium, in the case they are secreted into the medium, or from the cells of the microorganism and, if necessary, separated from other carotenoids that may be present in case one specific carotenoid is desired, by methods known in the art.

Carotenoids produced in accordance with the present invention can be used in a process for the preparation of food or feed. A man skilled in the art is familiar with such processes. Such compound food or feed can further comprise additives or components generally used for such purpose and known in the state of the art.

The following Examples further illustrate the present invention, but these are not thereby limiting the scope of the invention.

The following materials and methods were employed in the Examples described below:

### Strains

*P. rhodozyma* ATCC96594 (re-deposited under the accession No. ATCC 74438 on April 8, 1998 pursuant to the Budapest Treaty)
*P. rhodozyma* ATCC96815 (re-deposited under the accession No. ATCC 74486 on February 18, 1999 pursuant to the Budapest Treaty)
*E. coli* TOP10: F⁻, *mcrA,* delta(*mrr-hsd*RMS-*mcr*BC), phi80, delta(*lac*Z M15), delta(*lac*X74), *rec*A1, *deo*R, *ara*D139, (*ara-leu*)7697, *gal*U, *galK, rps*L (Str^{r}), *end*A1, *nup*G (Invitrogen Corporation, Carlsbad, USA)

### Vectors

pCR2.1-TOPO (Invitrogen Corporation, Carlsbad, USA)
pGEM-T (Promega Corporation, USA)

### Methods

Restriction enzymes and T4 DNA ligase were purchased from Takara Shuzo (Ohtsu, Japan).
Polymerase chain reaction (PCR) was performed with the thermal cycler from Perkin Elmer model 2400. Each PCR condition is described in examples. PCR primers were purchased from a commercial supplier. Fluorescent DNA primers for DNA sequencing were purchased from Pharmacia. DNA sequencing was performed with the automated fluorescent DNA sequencer (ALFred, Pharmacia).
An authentic sample for zeaxanthin and β-carotene was purchased from EXTRASYN-THESE S.A. (Genay Cedex, France) and WAKO (Osaka, Japan), respectively. β-cryptoxanthin was obtained from Roche Vitamins AG (Basle, Switzerland).

### Example 1: Cloning of the β-carotene hydroxylase gene (crtZ) from genomic DNA of Flavobacterium sp. ATCC21588

At first genomic DNA was prepared. Cells of *Flavobacterium* sp. R1534 WT (ATCC21588) (US 6,124,113) were inoculated into a 50 ml medium containing 1% glucose, 1% tryptone (Difco Laboratories), 1% yeast extract (Difco), 0.5% MgSO₄, and 3% NaCl, and cultivated at 27°C under aerobic condition. Overnight culture (50 ml) was centrifuged at 10,000 g for 10 min. The pellet was washed briefly with 10 ml of lysis buffer (50 mM EDTA, 0.1M NaCl, pH 7.5), resuspended in 4 ml of the same buffer supplemented with 10 mg of lysozyme and incubated at 37°C for 15 min. After addition of 0.3 ml of N-Lauroyl sarcosine (20%), incubation at 37°C was continued for another 15 min before extraction of DNA with phenol, phenol/chloroform and chloroform. The DNA was ethanol precipitated at room temperature for 20 min in the presence of 0.3 M sodium acetate (pH 5.2), followed by centrifugation at 10,000 g for 15 min. The pellet was rinsed with 70% ethanol, dried and resuspended in 1 ml ofTE (10 mM Tris, 1 mM EDTA, pH 8.0). Thus obtained genomic DNA was dialysed against H₂O for 48 hours using collodium bags (Sartorius, Germany), ethanol precipitated in the presence of 0.3 M sodium acetate and resuspended in H₂O. Using the prepared genomic DNA as template, a complete coding sequence for the β-carotene hydroxylase gene without excessive flanking region was obtained by PCR amplification using a thermal cycler (Perkin elmer 2400, USA). The synthetic primers used were: PZ1 (SEQ ID NO:1)(having a *Sma* I site CCCGGG) and PZ2 (SEQ ID NO:2)(having a *Bam*H I site GGATCC).
The PCR was performed using an Advantage-HF PCR Kit (CLONTECH Laboratories, Inc., USA). The PCR mixture was prepared according to the user's manual described by the manufacturer. The initial template denaturation step consisted of 1 min at 94°C. An amplification cycle of 30 seconds at 94°C and 4 min at 68°C was repeated for 25 times. After additional 3 min reaction at 68°C, the reaction mixture was kept at 15°C. By this reaction, DNA fragment containing a complete ORF of the β-carotene hydroxylase gene (522 bp) was amplified. This amplified β-carotene hydroacylase gene was ligated with a pCR2.1-TOPO vector and introduced into *E.coli* TOP 10 cells by using a TOPO TA Cloning kit (Invitrogen Corporation, USA) according to an instruction manual prepared by the manufacturer. Several clones were selected for sequence analysis. The sequence of the cloned β-carotene hydroxylase gene of each candidate clone was examined. One of the DNA clones that showed completely the same sequence as the β-carotene hydroxylase sequence of *Flavobacterium* sp. ATCC21588 (GenBank accession No. U62808) was named as pTOPO-PZ#23N. A 522 bp *Sma* 1 / *Bam*H I fragment cut out from pTOPO-PZ#23N was used as a crtZ gene cassette of Flavobacterium sp.

### Example 2: Cloning of the β-carotene hydroxylase gene (crtZ) from genomic DNA of E. herbicola ATCC39368

Cells of *E. herbicola (Escherichia vulneris*) ATCC39368 were inoculated into a 10 ml medium containing 1% tryptone (Difco Laboratories), 0.5% yeast extract (Difco), 0.5% NaCl, and cultivated at 26°C under aerobic conditions. Overnight culture (10 ml) was centrifuged at 10,000 g for 10 min. The pellet was used for the genomic DNA preparation using a QIAGEN Blood & Cell Culture DNA Midi Kit (QIAGEN, Germany) according to a protocol prepared by the manufacturer. Using the prepared genomic DNA as template, a complete coding sequence for the β-carotene hydroxylase gene without excessive flanking region was obtained by PCR amplification using a thermal cycler (Perkin elmer 2400, USA). The two synthetic primers used were EZ1 (SEQ ID NO:3)(having an *Sma* I site CCCGGG) and EZ2 (SEQ ID NO:4)(having an *Sal* I site GTCGAC).
The PCR was performed using an Advantage-HF PCR Kit (CLONTECH Laboratories, Inc., USA). The PCR mixture was prepared according to the user's manual described by the manufacturer. The initial template denaturation step consisted of 1 min at 94°C. An amplification cycle of 30 seconds at 94°C and 4 min at 68°C was repeated for 25 times. After additional 3 min reaction at 68°C, the reaction mixture was kept at 15°C. By this reaction, DNA fragment containing a complete ORF of the β-carotene hydroxylase gene (543 bp) was amplified. This amplified β-carotene hydroxylase gene was ligated with a pCR2.1-TOPO vector and introduced into *E.coli* TOP 10 cells by using a TOPO TA Cloning kit (Invitrogen Corporation, USA) according to an instruction manual prepared by the manufacturer. Several clones were selected for sequence analysis. The sequence of the cloned β-carotene hydroxylase gene of each candidate clone was examined. One of the DNA clones that showed completely the same sequence as the β-carotene hydroxylase sequence of *E. herbicola* ATCC39368 (GenBank accession No. M87280) was named as pTOPO-EZ#2. A 543 bp *Sma* I / *Sal* I fragment cut out from pTOPO-EZ#2 was used as a crtZ gene cassette of *E. herbicola.*

### Example 3: Preparation of components of the expression vector

The components of the expression vector, a G418 resistant gene, promoter and terminator region of glyceraldehyde-3-phosphate dehydrogenase gene (hereinafter referred to as GAP) of *P. rhodozyma*, and rDNA fragment of *P. rhodozyma* were prepared.
A G418 resistant gene cassette was prepared as follows. A *Sac* I- linker was ligated into the unique Hind III site of the vector, pUC-G418 (US Patent No. 6,365,386 B1), which is harboring the G418 resistant gene cassette, and resulted vector was named as pG418Sa512. A 1.7 kb *Kpn* I / *Sac* I fragment cut out from pG418Sa512 was used as a G418 resistant gene cassette.
Each of the promoter and the terminator of GAP gene and the rDNA fragment was obtained by PCR using the genomic DNA of *P. rhodozyma* ATCC 96594 as template. To obtain the genomic DNA, a QIAGEN Blood & Cell Culture DNA Midi Kit (QIAGEN, Germany) was used with the cells of *P. rhodozyma* ATCC 96594 obtained by overnight culture in YPD (Difco Laboratories) medium.
Using the prepared genomic DNA as a template, PCR was performed using an Advantage-HF PCR Kit (CLONTECH Laboratories, Inc., USA) and a thermal cycler (Perkin elmer 2400, USA).
The two synthetic primers used to amplify the promoter sequence of GAP were GAP#1 (SEQ ID NO:5)(having a *Not* I site GCGGCCGC) and GAP#5 (SEQ ID NO:6)(having a *Sma* I site CCCGGGG).
The initial template denaturation step consisted of 5 min at 94°C. An amplification cycle of 30 seconds at 94°C, 30 seconds at 55°C, and 1 min at 72°C was repeated for 25 times.
After additional 10 min reaction at 72°C, the reaction mixture was kept at 4°C. By this reaction, DNA fragment containing the GAP promoter (398 bp) was amplified. This amplified GAP promoter was ligated with a pCR2.1-TOPO vector and introduced into *E.coli* TOP 10 cells by using a TOPO TA Cloning kit (Invitrogen Corporation, USA).
Several clones were selected for sequence analysis. The sequence of the cloned GAP promoter of each candidate clone was examined. One of the DNA clones that showed completely the same sequence as the GAP promoter of *P. rhodozyma* (GenBank accession No. Y08366) was named as pTOPO-pGAP2#1. A 398 bp *Not* I / *Sma* I fragment cut out from pTOPO-pGAP2#1 was used as the GAP promoter cassette.
The synthetic primers used to amplify the terminator sequence of GAP were GAP#33 (SEQ ID NO:7)(having a *Bam*H I-*Sal* I site GGATCCGTCGAC)and GAP#4 (SEQ ID NO:8) (having a *Kpn* I siteGGTACC).

The PCR conditions were the same as those for the GAP promoter described above. By this reaction, DNA fragment containing the GAP terminator (302 bp) was amplified. This amplified GAP terminator was ligated with a pCR2.1-TOPO vector and introduced into *E.coli* TOP 10 cells by using the TOPO TA Cloning kit. Several clones were selected for sequence analysis. The sequence of the cloned GAP terminator of each candidate clone was examined. One of the DNA clones that showed completely the same sequence as the GAP terminator of *P. rhodozyma* (GenBank accession No. Y08366) was named as pTOPO-tGAP#1. A 302 bp *BamH* I / *Kpn* I fragment or a 296 bp *Sal* I / *Kpn* I fragment which was cut out from pTOPO-tGAP#1 was used as the GAP terminator cassette.
The two synthetic primers used to amplify the rDNA fragment were R#1 (SEQ ID NO:9) (having a *Sac* I site GAGCTC) and R#2 (SEQ ID NO:10)(having a *Not* I-*Sac* I site GCGGCCGCGAGCTC).
The PCR conditions were the same as those for the GAP promoter described above. By this reaction, DNA fragment containing the rDNA (3126 bp) was amplified. This amplified the rDNA was ligated with a pCR2.1-TOPO vector and introduced into *E.coli* TOP 10 cells by using the TOPO TA Cloning kit. Several clones were selected for sequence analysis. The sequence of the cloned the rDNA of each candidate clone was examined. One of the DNA clones that showed completely the same sequence as the rDNA of *P. rhodozyma* (GenBank accession No. D31656, AF139632) was named as pTOPO-rDNA#1. A 1960 bp *Sac* II / *Not* I fragment cut out from pTOPO-rDNA#1 was used as the rDNA cassette.

### Example 4: Construction of the expression vector carrying the β-carotene hydroxylase gene (crtZ) obtained from Flavobacterium sp. or E. herbicola

To construct the expression vector carrying the crtZ of *Flavobacterium* sp., the rDNA cassette and the GAP promoter cassette (obtained in Example 3), the crtZ gene cassette of *Flavobacterium* sp. (obtained in Example 1), and the *BamH* I / *Kpn* I fragment of the GAP terminator cassette and the G418 resistant gene cassette (obtained in Example 3) were aligned by ligation in this order using a pGEM-T as a backbone. The resulting expression vector was named as pRPZG-ptGAP2.
To construct the expression vector carrying the crtZ of *E. herbicola,* the rDNA cassette and the GAP promoter cassette (obtained in Example 3), the crtZ gene cassette of *E. herbicola* (obtained in Example 2), and *Sal* I / *Kpn* I fragment of the GAP terminator cassette and the G418 resistant gene cassette (obtained in Example 3) were aligned by ligation in this order using a pGEM-T as a backbone. Resulted expression vector was named as pREZG-ptGAP2.

### Example 5: Introduction of the expression vector carrying the β-carotene hydroxylase gene (crtZ) of Flavobacterium sp. or E. herbicola into P. rhodozyma

Each of the expression vectors obtained in the Example 4, pRPZG-ptGAP2 and pREZG-ptGAP2, carrying the *crtZ* gene of *Flavobacterium* sp. and *E. herbicola,* respectively, was introduced into *P. rhodozyma* ATCC 96815, a mutant strain producing β-carotene predominantly by using the particle-gun method as described in EP 1,158,051. YPD-agar medium containing 0.75 M D-sorbitol and D-mannitol, and 0.1 mg/ml of geneticin was used for direct selection of the recombinant strains.
As a result, hundreds of transformants which were resistant to 0.1 mg/ml geneticin were obtained by each of pRPZG-ptGAP2 and pREZG-ptGAP2, and 35 each transformants were selected randomly for further characterization.

### Example 6: Characterization of the crtZ-recombinant strains of P. rhodozyma

The *crtZ*-recombinant strains of *P. rhodozyma* ATCC 96815 thus obtained were cultivated in 50 ml production medium in 500 ml Erlenmeyer flask with shaking at 20°C for 7 days after inoculation of seed culture prepared in 7 ml seed medium in test tube (21 mm in diameter) with shaking at 20°C for 3 days. Appropriate volume of the culture broth was withdrawn and used for analysis of the cell growth and the productivity of carotenoids. Medium composition was as follows:
Seed medium: Glucose30.0 g/l, NH₄Cl 4.83 g/l, KH₂PO₄ 1.0 g/l, MgSO₄-7H₂O 0.88 g/l, NaCl 0.06 g/l, CaCl₂-2H₂O 0.2 g/l, KH phtalate 20.0 g/l, FeSO₄-7H₂O 28 mg/l, Trace element solution 0.3 ml, Vitamin stock solution 1.5 ml, (pH was adjusted at 5.4-5.6). Trace element solution: 4N- H₂SO₄ 100 ml/l, citric acid-H₂O 50.0 g/l, ZnSO₄-7H₂O 16.7 g/l, CuSO₄-5H₂O 2.5 g/l, MnSO₄-4,5H₂O 2.0 g/l, H₃BO₃ 2.0 g/l, Na₂MoO₄ 2.0 g/l, KI 0.5 g/l.
Vitamin stock solution for seed medium: 4N- H₂SO₄ 17.5 ml/l, *myo*-Inositol 40.0 g/l, Nicotinic acid 2.0 g/l, Ca-D-pantothenate 2.0 g/l, Vitamin B₁ (thiamin HCl) 2.0 g/l, *p-*Aminobenzoic acid 1.2 g/l, Vitamin B₆ (pyridoxine HCl) 0.2 g/l, Biotin stock solution 8.0 ml.
Biotin stock solution was prepared by the addition of 4N- H₂SO₄ to 50 ml of ethanol to a total of 100 ml. Then, 400 mg of D-biotin were added.
Production medium: Glucose 22.0 g/l, KH₂PO₄ 14.25 g/l, MgSO₄-7H₂O 2.1 g/l, CaCl₂-2H₂O 0.865 g/l, (NH₄)₂SO₄ 3.7 g/l, FeSO₄-7H₂O 0.28 g/l, Trace element solution 4.2 ml, Vitamin stock solution 9.35 ml, (pH was adjusted at 5.5).
Vitamin stock solution for production medium: 4N- H₂SO₄ 17.5 ml/l, Nicotinic acid 2.0 g/l, Ca-D-pantothenate 3.0 g/l, Vitamin B₁ (thiamin HCl) 2.0 g/l, p-Aminobenzoic acid 1.2 g/l, Vitamin B₆ (pyridoxine HCl) 0.2 g/l, Biotin stock solution 30.0 ml
A portion of the seed culture broth (2.5 ml) was transferred to 47.5 ml of the production medium in 500 ml Erlenmeyer flask. Then the cultivation was started at 20°C with rotary shaking at 200 rpm. At the second day of the fermentation, 5 ml of 50 % glucose solution was added to the medium and then the fermentation was continued. At the fourth day of the fermentation, 2 ml of cultured broth was withdrawn and then 5 ml of 50 % glucose solution was added again to the medium, and cultivation was continued for additional 3 days. At the seventh day, an aliquot of the culture was withdrawn and used for analysis of the carotenoids production and the cell growth.
For analysis of the cell growth, optical density at 660 nm was measured by using UV-1200 photometer (Shimadzu Corp., Kyoto, Japan).

For analysis of the content of β-carotene, β-cryptoxanthin and zeaxanthin, the withdrawn broth was mixed with a solvent mixture (ethyl alcohol, hexane and ethyl acetate) and carotenoids were extracted from the broth and the cells of *P. rhodozyma* by vigorous shaking with glass beads. After extraction, disrupted cells and glass beads were removed by centrifugation and the resultant supernatant was analyzed by HPLC for the carotenoids content. The HPLC conditions used is as follows: HPLC column: Chrompack Lichrosorb si-60 (4.6 mm, 250 mm); Temperature: room temperature; Eluent: acetone / hexane (18/82) add 1 ml / 1 of water to eluent; Injection volume: 10 µl, Flow rate: 2.0 ml / minute; Detection: UV at 450 nm.
As a result, it was found that all recombinant strains (35 each transformants for two kinds of *crfZ*-expression vectors, pRPZG-ptGAP2 and pREZG-ptGAP2) produced zeaxanthin and β-cryptoxanthin without accumulation of astaxanthin.
Fermentation profile of the representative 3 each recombinant strains selected from the transformants expressing *crtZ* gene of Flavobacterium sp. or *crtZ* gene of *E. herbicola,* respectively, is shown in the Table together with that of their host strain (*P. rhodozyma* ATCC 96815).

**Table: Carotenogenesis and growth profile of the recombinant strains in shake flask culture**

| Strain | Origin of *crtZ* | CARO (mg/l) | CRYPTO (mg/l) | ZEA (mg/l) | ASTA (mg/l) | OD at 660 nm |
|---|---|---|---|---|---|---|
| PZ-7 | *Flavobacterium* sp. | 4.7 | 3.5 | 5.7 | 0.0 | 46.3 |
| PZ-24 | *Flavobacterium* sp. | 7.1 | 2.7 | 2.7 | 0.0 | 48.3 |
| PZ-25 | *Flavobacterium* sp. | 6.3 | 2.1 | 2.7 | 0.0 | 48.3 |
| EZ-7 | *E. herbicola* | 3.3 | 2.7 | 2.2 | 0.0 | 49.0 |
| EZ-13 | *E. herbicola* | 3.3 | 2.8 | 2.3 | 0.0 | 44.7 |
| EZ-14 | *E. herbicola* | 3.6 | 3.1 | 2.4 | 0.0 | 45.9 |
| ATCC 96815 | - | 10.2 | 0.0 | 0.0 | 0.0 | 51.9 |

CARO: β-carotene; CRYPTO: β-crypto-xanthin; ZEA: Zeaxanthin; ASTA: Astaxanthin As shown in the Table, conversion from β-carotene into β-cryptoxanthin and zeaxanthin was exemplified in all the *Phaffia* recombinant strains expressing crtZ gene of *Flavobacterium* sp. or crtZ gene of *E. herbicola.*

## Claims

1. A process for producing zeaxanthin and β-cryptoxanthin which comprises cultivating a recombinant microorganism which is expressing a β-carotene hydroxylase gene and belonging to the genus *Xanthophyllomyces (Phaffia)* in an aqueous nutrient medium under aerobic conditions, and isolating the resulted carotenoids from the cells of said recombinant microorganism or from the cultured broth, wherein the β-carotene hydroxylase gene is originated from a microorganism which is selected from the group consisting of *Flavobacterium* sp.R1534 WT (ATCC21588), *Erwinia uredovora* ATCC19321, *Erwinia herbicola* ATCC39368, *Agrobacterium aurantiacum, Alcaligenes* PC-1, *Paracoccus marcusii* MH1, and gram-negative bacteria E-396 (FERM BP-4283) which are having the β-carotene hydroxylase gene and wherein the cultivation is carried out at pH range from 4 to 8 and at a temperature range from 15 to 26°C for 24 to 500 hours.

2. The process according to claim 1, wherein the recombinant microorganism is derived from *Xanthophyllomyces dendrorhous (Phaffia rhodozyma)* ATCC96815.

3. The process according to claim 1 or 2, wherein the β-carotene hydroxylase gene is originated from *Flavobacterium* sp.R1534 WT (ATCC21588) or the DNA sequence of the β-carotene hydroxylase gene is substantially homologous thereto, whereby the amino acid sequence thereof shows more than 90 % identical amino acids when compared to the amino acid sequence of crtZ of *Flavobacterium* sp.R1534 WT.

4. The process according to any one of claims 1 to 3, wherein the β-carotene hydroxylase gene is expressed in the recombinant microorganism using the control sequences which are capable of effecting the expression of DNA sequences in a microorganism belonging to Phaffia.

5. The process according to claim 1, wherein the cultivation is carried out at pH range from 5 to 7 and at a temperature range from 18 to 22°C for 48 to 350 hours.

## Patentansprüche

1. Verfahren zum Herstellen von Zeaxanthin und β-Cryptoxanthin, das das Kultivieren eines rekombinanten Mikroorganismus, der ein β-Carofin-hydroxylase-gen exprimiert und der der Gattung *Xanthophyllomyces* (*Phaffia*) angehört, in einem wässrigen Nährmedium unter aeroben Bedingungen und das Isolieren der resultierenden Carotinoide aus den Zellen des rekombinanten Mikroorganismus oder aus der Kulturbrühe umfasst, wobei das β-Carotin-hydroxylase-gen aus einem Mikroorganismus stammt, der ausgewählt ist aus der Gruppe, bestehend aus *Flavobacterium* sp.R1534 WT (ATCC21588), *Erwinia uredovora* ATCC19321, *Erwinia herbicola* ATCC39368, *Agrobacterium aurantiacum, Alcaligenes* PC-1, *Paracoccus marcusii* MH1 und einem gram-negativen Bakterium E-396 (FERM BP-4283) die das β-Carotin-hydroxylase-gen haben, und wobei das Kultivieren in einem pH-Bereich von 4 bis 8 und in einem Temperaturbereich von 15 bis 26°C für 24 bis 500 Stunden durchgeführt wird.

2. Verfahren nach Anspruch 1, wobei der rekombinante Mikroorganismus aus *Xanthophyllomyces dendrorhous* (*Phaffia rhodozyma)* ATCC96815 abgeleitet ist.

3. Verfahren nach Anspruch 1 oder 2, wobei das β-Carotin-hydroxylase-gen aus *Falvobacterium* sp.R1534 WT (ATCC21588) stammt oder die DNA-Sequenz des β-Carotin-hydroxylase-gens im Wesentlichen homolog dazu ist, wobei die Aminosäuresequenz davon mehr als 90% identische Aminosäuren zeigt, wenn sie mit der Aminosäuresequenz von crtZ von *Flavobacterium* sp.R1534 WT verglichen wird.

4. Verfahren nach einem der Ansprüche 1 bis 3, wobei das β-Carotin-hydroxylase-gen in dem rekombinanten Mikroorganismus unter Verwendung der Kontrollsequenzen exprimiert wird, die die Expression von DNA-Sequenzen in einem zu Phaffia gehörigen Mikroorganismus bewirken können.

5. Verfahren nach Anspruch 1, wobei die Kultivierung in einem pH-Bereich von 5 bis 7 und in einem Temperaturbereich von 18 bis 22°C für 48 bis 350 Stunden durchgeführt wird.

## Revendications

1. Procédé de production de zéaxanthine et de β-cryptoxanthine qui comprend les étapes consistant à :
- cultiver un microorganisme recombinant qui exprime un gène de l'hydroxylase de β-carotène et appartient au genre *Xanthophyllomyces (Phaffia)* dans un milieu nutritif aqueux en conditions aérobie, et
- isoler les caroténoïdes résultants des cellules dudit microorganisme recombinant ou du bouillon de culture, dans lequel le gène de l'hydroxylase de β-carotène provient d'un microorganisme qui est sélectionné à partir d'un groupe constitué de *Flavobacterium* sp. R1534 WT (ATCC21588), *Erwinia uredovora* ATCC19321, *Erwinia herbicola* ATCC39368, *Agrobacterium aurantiacum, Alcaligenes* PC-1, *Paracoccus marcusii* MH1, et des bactéries gram-négatif E-396 (FERM BP-4283) qui ont le gène de l'hydroxylase de β-carotène et dans lequel la culture est réalisée dans une gamme de pH allant de 4 à 8 et dans une gamme de température allant de 15°C à 26°C pendant 24 à 500 heures.

2. Procédé selon la revendication 1, dans lequel le microorganisme recombinant est dérivé de *Xanthophyllomyces dendrorhous* (*Phaffia rhodozyma)* ATCC96815.

3. Procédé selon la revendication 1 ou 2, dans lequel le gène de l'hydroxylase de β-carotène provient de *Flavobacterium* sp. R1534 WT (ATCC21588) ou la séquence d'ADN du gène de l'hydroxylase de β-carotène y est substantiellement homologue, dans lequel la séquence d'acides aminés de celui-ci présente plus de 90% d'acides aminés identiques lorsqu'on les compare à la séquence d'acides aminés de crtZ de *Flavobacterium* sp. R1534 WT.

4. Procédé selon l'une des revendications 1 à 3, dans lequel le gène de l'hydroxylase de β-carotène est exprimé dans le microorganisme recombinant en utilisant les séquences contrôle qui sont capables d'effectuer l'expression des séquences d'ADN dans un microorganisme appartenant à Phaffia.

5. Procédé selon la revendication 1, dans lequel la culture est réalisée dans une gamme de pH allant de 5 à 7 et dans une gamme de température allant de 18 à 22°C pendant 48 à 350 heures.
